# EUROPEAN PATENT SPECIFICATION

(11) **EP 1 882 629 B1**
(45) Date of publication and mention of the grant of the patent: **02.07.2014**
(21) Application number: 07022378.9
(22) Date of filing: 10.08.2001
(51) Int. Cl.: B65B 3/00, B65B 7/28, B65B 9/02

(54) **Method for filling syringes**
Verfahren zum Befüllen von Spritzen
Procédé pour le remplissage de seringues

(30) Priority: 10.08.2000 US 224136 P
(43) Date of publication of application: 30.01.2008
(62) Divisional of application: 01970534.2
(73) Proprietor: Baxa Corporation, Englewood, Colorado 80112-3093 (US)
(72) Inventor: Baldwin, Brian Eugene, Centennial CO 80016 (US); Mancuso, Robert Eugene, Elizabeth CO 80107 (US)
(74) Representative: Mallalieu, Catherine Louise

(56) References cited:
- EP-A2- 0 227 401
- EP-B1- 0 961 733
- WO-A1-98/33705
- US-A- 3 823 818

## Description

### RELATED APPLICATIONS

This application claims priority under 35 U.S.C. § 119 to prior U.S. Patent Application Serial No. 60/224,136, filed August 10, 2000, the entirety of which is hereby incorporated by reference.

### FIELD OF THE INVENTION

The p>resent invention generally relates to the handling of syringes, and is particularly apt for use in automated syringe handling operations, such as syringe filling, labeling and capping operations.

### BACKGROUND OF THE INVENTION

Each year countless syringes are used throughout the world by the healthcare industry for the administration of liquid medications to humans and animals with hypodermic needles or infusion catheters, as well as for delivery of oral and topical medications. Some medications provided by pharmaceutical manufacturers are prepared, stored, and shipped as powders, crystals, or some other solid form due to the lack of stability in solution. These medications are then reconstituted with liquid, such as water or some other suitable liquid solvent. For one or several administrations of a medication, the manual filling of the syringes with reconstituted liquid medication is a small chore. However, larger health care institutions often administer medications in syringes to hundreds of patients per day, thus requiring the rather large chore of filling hundreds of syringes with medications and labeling each filled syringe to show the contents, strength, and fill dates, usually under the direction of a qualified pharmacist. Healthcare providers have found that preparing (e.g. filling and labeling) the quantities of syringes needed has many efficiencies and other advantages when it is done in batches.

In the later regard, batch preparation may be particularly preferred for syringes carrying medications that are not stable in liquid form and are therefore frozen after preparation to maintain acceptable stability. Further, the task of maintaining sterility in the transfer of liquid from containers provided by pharmaceutical manufacturers to pre-sterilized syringes may be enhanced by batch completion in controlled environments. Also, safety and overall reliability may improve when syringes are prepared in batches by pharmacy personnel or others who are dedicated to and well-trained for the task.

Currently, syringe preparation typically entails a number of separate operations with individual syringe handling. For example, systems used today fill syringes with dispensing pumps that are capable of delivering exact quantities of fluids but that require individual handling of each syringe. Peristaltic pumps that can be accurately calibrated, such as that described in U.S. Patent No. 5,024,347, are often used. In such arrangements, The syringe caps are packaged so that sterility can be maintained in the capping procedure. The caps are located in trays where each cap is positioned so that the person doing the filling can manually place the tip of the syringe into the cap without touching or holding the cap. Labeling of the syringes has been done using a label dispenser similar to those used for applying pricing labels to grocery or other similar products.

With smaller syringes there are sometimes problems with getting sufficient label information on the syringe without covering over the syringe graduations or blocking the view of the medication. To overcome this, the labels are often applied by hand with the label wrapped around the syringe with most of the label extending from the syringe to form a flag.

Silicone lubricants are used in syringe manufacturing to provide lubrication for lowering the frictional force in movement of the syringe plunger. These silicone lubricants have a characteristic of migrating over all surfaces. Often, this migration causes difficulties in getting pressure sensitive labels to stay in place. This has caused users to use a clear plastic tape to wrap completely around the syringe and the label.

Efforts to automate hospital or clinic-based syringe preparation have been made, but most systems have automated only portions of the process and still require human intervention during critical stages of the process. In one such system, caps are pre-positioned in a cartridge holder. The syringes are also provided in a cartridge where each syringe is oriented. The machine to perform the filling and capping function requires an operator to load the cartridges of caps and syringes. The filling is done with a calibrated peristaltic pump. The machine fills each syringe and places a cap. The labeling is done separately by a labeling machine that is commercially available.

WO 98/33705 A1 discloses a system for automatically producing a plurality of prefilled, sterile delivery devices with a desired quantity of fluid therein.

EP 0 961 733 B1 relates to a method of handling, filling and sealing prefabricated packaging containers provided with a closure device.

US 3 823 818 A discloses a method for handling and storing preforms which are to be blow molded into finished containers.

EP 0 227 401 A2 discloses a process for producing sterilised prefilled plastic syringes avoiding plastic deformation and distortion.

### SUMMARY OF THE INVENTION

In view of the foregoing, a broad objective of the present invention is to provide a method for enhanced syringe handling. Also described herein is a interconnecting a liquid dispensing apparatus with a dispensing end of the syringe body in an automated manner; and flowing fluid into the syringe body from the liquid dispensing apparatus, through the dispensing end of the syringe body, by drawing the fluid into the syringe body by retraction of a plunger relative to the syringe body. A closely related objective is to facilitate automated syringe handling for various operations, such as syringe filling, labeling and capping.

Another objective of the present invention is to provide a syringe handling approach that facilitates the maintenance of sterility.

An additional objective of the present invention is to provide an improved syringe filling and capping approach.

Yet another objective of the present invention is to provide an improved approach for syringe labeling.

In addressing one or more of the above objectives, the present inventors have recognized that significant benefits may be realized by interconnecting multiple syringe bodies to facilitate handling of the same. More particularly, such interconnection allows multiple syringes to be commonly oriented for packaging and/or automated preparation operations.

An apparatus is provided that includes a plurality of syringe bodies, e.g. each comprising a barrel, and a belt fixedly connected to (e.g. adhered to or shrink-wrapped upon) each of the syringe bodies. Each syringe body may further include a plunger at least partially disposed in an open end of the barrel and a removable cap disposed on a dispensing end of the barrel. Of importance, the belt is provided to both interconnect the plurality of syringe bodies and position the same in a predetermined orientation.

In the later regard, and by way of primary example, the dispensing ends of the syringe body barrels may be oriented to extend in a common direction. In addition, the barrels of adjacent ones of the plurality of syringe bodies may be disposed in side-by-side, series relation. Further, the belt may be provided to define a predetermined spacing between adjacent ones of the syringe bodies, such spacing preferably being equidistance throughout a given assembly to accommodate ready positioning in holders adapted for automated operations, as will be further described.

To facilitate handling, production and packaging, the belt may be of a pliable construction. Further, the belt may be advantageously constructed for ready separation in automated labeling operations, as described hereinbelow. In this regard, it is advantageous for the belt to be of a predetermined length between adjacent ones of the plurality of syringe bodies, such predetermined length defining belt segments that are sufficient for the placement of contents information thereupon(e.g. via the application of a label thereto or direct printing thereupon).

Preferably, the belt is interconnected to each of the syringe body barrels. In this regard, the barrels maybe of a common length, wherein the belt is fixedly connected to the barrels along a common portion of the length of each. In addition, the belt may advantageously be of a width that exceeds a majority of a length of each of the barrels. Further, the belt may comprise a first portion that extends between adjacent ones of the plurality of syringe bodies, and a second portion that extends about at least a portion of each of the syringe body barrels. Preferably, the second portion adhesively engages the syringe body barrels and may be substantially transparent to facilitate observation of the volumetric contents within and markings on the syringe barrels.

In one approach, the belt may be defined by opposing layers adjoined in face-to-face relation between adjacent ones of the plurality of syringe bodies and wrapped about opposing sides of the barrels of each of the syringe bodies. At least one of the opposing layers may be substantially transparent to allow for visual determination of volumetric contents and amount. As may be appreciated, a clear pliable plastic material may be utilized for easy and low-cost construction of the belt.

As noted, each syringe body of the apparatus may typically include a plunger and cap. In this regard, the barrel, inserted plunger and applied cap may preferably be assembled under low bioburden environment conditions, such as a class 100,000 or lower clean room. Further, and of importance, the plurality of interconnected syringe bodies should preferably be packaged (e.g. in a shipment container) and thereafter sterilized (e.g. via gamma radiation) to achieve terminal sterilization.

To facilitate the maintenance of a clean internal volume, yet allow for syringe filling, the caps utilized on syringe bodies should preferably engage dispensing ends of the barrels in a mating fashion. By way of primary example, each cap may include an inner member matingly positionable within or about a fluid port of the barrel

dispensing end, and an outer member matingly positionable about an outer flange of the barrel dispensing end.

A method also is provided for producing an assembly of syringe bodies. The method includes the steps of positioning a plurality of syringe bodies in a predetermined relative orientation, and disposing opposing layers of material about opposing sides of the syringe bodies and in face-to-face relation between adjacent ones of the syringe bodies. As may be appreciated, the method defines an assembly comprising a belt that interconnects and orients a plurality of syringe bodies to facilitate handling as previously described.

In an additional more general aspect, an overall method and apparatus for handling a plurality of syringe bodies is provided. Such method comprises the steps of positioning a plurality of syringe bodies in a predetermined orientation, and interconnecting a belt to each of the plurality of syringe bodies in said predetermined orientation. The method may further comprise the step of positioning the plurality of syringe bodies into a plurality of holders for at least one production operation. To facilitate such positioning, the belt may advantageously define a predetermined spacing between adjacent ones of the syringe bodies, wherein the holders are separated by a distance that corresponds with the predetermined spacing between adjacent ones of the syringe bodies. Further, where the belt is constructed of a pliable material, the method may include the step of successively suspending, or hanging, adjacent ones of the syringe bodies so as to position the same for receipt by a holder.

Numerous automated production operations may be facilitated by the disclosed handling method, wherein the holders may be moved along a predetermined path during such operations. Of particular note, one or all of the following production operations may be automated utilizing the disclosure herein:
filling the plurality of syringe bodies with a predetermined fluid (e.g.
   reconstituted medication);
uncapping and/or recapping the plurality of syringe bodies in conjunction with filling; and labeling the plurality of the syringe bodies to indicate the contents thereof.

Each of these production operations will be further described hereinbelow.

In relation to the apparatus for handling a plurality of syringe bodies, it should be appreciated that it is particularly advantageous for the syringe bodies to be interconnected in series by a belt in a predetermined orientation and with a predetermined spacing therebetween. In the latter regard, the apparatus may comprise a plurality of holders for holding the of syringe bodies, such holders being separated by a distance corresponding with the predetermined spacing.

The apparatus may further include a drive for moving the holders along a predetermined path. In this regard, the holders may be oriented so as to locate adjacent ones of the plurality of syringe bodies in substantial parallel relation, wherein the dispensing and opposing ends of the syringe bodies extend outwardly from and in a common orientation relative to the predetermined path. In turn, at least one workstation may be provided having a support member disposed to move towards and away from the dispensing ends of the syringe bodies. By way of primary example, such workstations may be provided for automated filling and/or automated cap removal/replacement, free from manual handling requirements.

Further, one or more workstations may be provided with a support member disposed to move towards and away from an outward facing surface of the belt at locations between adjacent ones of the syringe bodies. Such workstations may provide for automated separation of the belt between adjacent ones of the syringe bodies and/or automated printing of contents information on belt segments located between adjacent ones of the syringe bodies.

The present invention provides a method for filling a plurality of syringe bodies. Also described is an apparatus for filling syringe bodies. In the inventive method, the filling of each syringe body entails the step of holding the syringe body in at least one holder and the further steps of removing a cap from, filling and replacing the cap back on the syringe body during the holding step. As may be appreciated, completion of the removing, filling and replacing steps while the syringe body is being held by at least one holder yields a significant handling advantage in that manual manipulation of a syringe body may be avoided. The filling step includes interconnecting a liquid dispensing apparatus (420) with a dispensing end of the syringe body (S) in an automated manner; and flowing fluid into the syringe body (S) from the liquid dispensing apparatus (420), through the dispensing end of the syringe body(S), by drawing the fluid into the syringe body (S) by retraction of a plunger (P) relative to the syringe body (S).

The filling method may further include, for each syringe body, the steps of placing the cap on the dispensing end of the syringe body prior to the holding step, and packaging the syringe body in a container (e.g. for bulk shipment with other syringe bodies) and unpackaging the syringe body from the container after the placing step and prior to the holding step. Such sequencing allows for cap placement and
packaging in a production location, followed by shipment to a remote location for unpackaging and completion of the filling method. Further in this regard, the method may include the important step of sterilizing syringe bodies after packaging (e.g. at the production facility prior to shipment).

Additionally, the method may comprise the step of interconnecting a belt to the plurality of syringe bodies in a predetermined orientation. Preferably, such interconnection occurs prior to the packaging and sterilization steps.

In conjunction with the removal and replacement of each of the caps, such steps may include, for each of the syringe bodies, the further steps of retainably engaging the cap in a retainer and moving at least one of the retainer and the holder to affect relative movement between the cap and the dispensing end of the syringe body. Further in this regard, such retainable engagement may be completed by moving the holder for a syringe along a predetermined path so as to insert the cap in the retainer.

In conjunction with noted filling step, the method further provide for the interconnection of a fluid supply member (also termed liquid dispensing apparatus) with a dispensing end of the syringe body and for the flow of fluid into the syringe body through the interconnected fluid supply member. In one embodiment, such steps as well as the cap removal and cap replacement steps, may be completed with the syringe body held at a single location. In such embodiment the retainer, and fluid supply member may be interconnected for tandem forward/rearward and sideways movement. In another embodiment, the cap removal and cap replacement steps may be completed with a syringe body held at a first location, while the filling step may be completed at a second location. Such an approach only requires forward/rearward tandem movement of the retainer and fluid supply member. In one embodiment, the liquid dispensing apparatus includes a valve, and for each syringe body the filling step comprises: retracting a plunger flange retention member away from the syringe body with the valve open; and, closing the valve after the retracting step.

Of note, the inventive filling method and disclosed apparatus may also provide for sensing of the position of a syringe body plunger during fluid filling. In this regard, optical sensing, pressure sensing or the like may be utilized, wherein a sense signal may be provided that reflects the fluid volume within a syringe as it is filled. In turn, the sense signal may be employed to terminate the flow of fluid at a predetermined amount. In another approach, a predetermined amount of fluid may be drawn into each syringe body via controlled retraction of the associated plunger.

As may be appreciated, the apparatus for filling a plurality of syringe bodies may include at least one, and preferably a plurality of holders for holding a plurality of syringe bodies in a predetermined orientation. Further, the apparatus may include a retainer for retainably engaging the cap of a syringe body, wherein the cap may be selectively removed and replaced by the retainer. Additionally, the apparatus includes a fluid supply member disposed for selective fluid interconnection with a dispensing end of the syringe body.

To facilitate automated operations, the apparatus may further comprise a driven support member for moving the holder(s) along a predetermined path. Additionally, one or more driven support members may be provided for moving the retainer towards/away from the dispensing end(s) of each syringe body and/or for moving the fluid supply member towards and away from the dispensing end(s) of each syringe body.

A method and apparatus are also provided for labeling a plurality of syringe bodies. The method includes the steps of interconnecting a belt to a plurality of syringe bodies in a predetermined orientation, and placing contents-related information on belt segments interconnected to each of the syringe bodies. The method further includes the step of separating the belt between each of said plurality of syringe bodies to define an interconnected flap (e.g. corresponding with the belt segments) on each of the syringe bodies.

In conjunction with the labeling method, the separating step may provide for severing, or cutting the belt between adjacent ones of the plurality of syringe bodies. Alternatively, the separating step may entail relative displacement of adjacent ones of the syringe bodies so as to achieve separation along perforation lines or the like.

With respect to the step of placing contents-related information on each given belt segment, such step may entail the printing of information on a label and fixation of such label to a belt segment. Alternatively, this step may simply be completed via printing of the contents-related information directly on a given belt segment.

In either case, the contents-related information may comprise one or more of the following types of information:
information regarding the fluid contained in a given syringe body;
information regarding fluid fill date for each given syringe body;
information regarding the volumetric fluid content of each given syringe body;
information comprising a product code corresponding with the contents of a given syringe body;
information regarding the lot or batch number corresponding with each given syringe body; and
information regarding storage and/or handling instructions for each given syringe body.

As may be appreciated, such information may be provided in an alphanumeric or coded fashion. In the later regard, at least some of the information may be embodied in a bar code format to allow for optical scanning.

In further relation to the labeling method, the interconnected syringe
bodies
may be packaged in a container, sterilized and unpackaged from the container prior to the separating and contents-information placement steps. As may be appreciated, such sequencing provides for the interconnection, packaging and sterilization of syringe bodies at a production location, and the unpackaging, separation and labeling of the syringe bodies at another location (e.g. at a location where the syringe bodies are filled with liquid medication).

The labeling apparatus is particularly adapted for use with a plurality of syringe bodies interconnected by belt, as described above, and may include a plurality of holders and a labeling member for placing contents-related information on belt segments extending between the syringe bodies. The apparatus may further include a separation member for separating the belt between adjacent ones of the plurality of syringe bodies, wherein a different belt segment in the form of a flap is interconnected with each one of the plurality of syringe bodies. To facilitate operation of the separation member and labeling member, each of such members may be provided with driven support members that may be selectively actuated to such members towards and away from the belt segments.

As may be appreciated, various ones of the methods and apparatuses noted hereinabove may be combined to yield a system for handling a plurality of syringe bodies, including a system that facilitates automated labeling and filling operations. The automated filling operations may further provide for automated cap removal replacement.

These and other aspects, advantages, and novel features of the invention and the disclosed methods and apparatuses are set forth in part in the description that follows and will become apparent to those skilled in the art upon examination of the following description and figures or may be learned by practicing the invention.

### BRIEF DESCRIPTION OF THE DRAWINGS

The accompanying drawings, which are incorporated in and form a part of the specification, illustrate embodiments of the present invention, and the methods and apparatuses disclosed herein, together with the descriptions serve to explain the principles thereof.
Figure 1 is an isometric view of a labeled, filled, and capped syringe with a label substrate and label attached;
Figure 2 is an isometric view of a plurality of sterile capped syringes mounted in a belt or band for automated labeling and/or cap removal, fluid filling, and cap replacement;
Figure 3 is a diagrammatic elevation view of an apparatus and process for mounting syringes in a tape band or belt;
Figure 4 is diagrammatic elevation view of an apparatus and process for mounting syringes in a tape band or belt;
Figure 5 is a diagrammatic elevation view of a labeling and filling apparatus;
Figures 6a through 6e comprise diagrammatic plan views of the syringe-filling station on the apparatus of Figure 5 wherein a sequence of component positions are shown that correspond to and illustrate sequential steps of cap removal, fluid filling, and cap replacement operation.
Figures 7a and 7b comprise isometric assembly and exploded views, respectively, of a labeling and filling apparatus corresponding with Figures 5 and 6a-e;
Figures 8a-8d comprise isometric views of the syringe-filling station of the apparatus of Figure 7, wherein a sequence of component positions are shown that correspond with and illustrate the sequential steps of cap removal, fluid filling, and cap replacement operations.
Figure 9 is a schematic elevation view of a labeling and filling apparatus;
Figure 10 is an isometric view of a syringe-filling station of the apparatus of Figure 9;
   and
Figures 11a-11h are flat, diagrammatic views of syringe handling operations at the filling-station of the apparatus of Figures 9 and 10.
Figures 12a-12c are isometric, end and cross-sectional views of a syringe cap employable in the syringe shown in Figure 1.
Figures 13a-13c are isometric, end and cross-sectional views of a syringe cap employable in another version of the syringe shown in Figure 1.

### DETAILED DESCRIPTION OF THE INVENTION

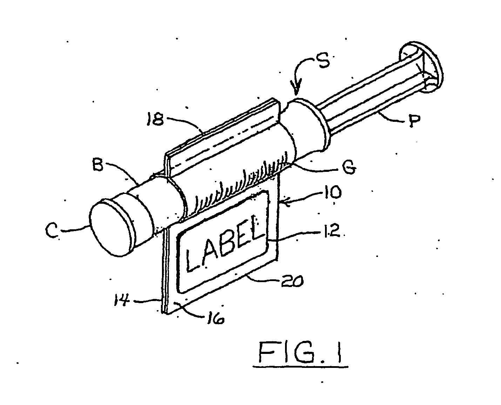

A capped syringe S that has been labeled and filled according to methods described herein is shown in Figure 1. A cap C covers and protects the sterility of the dispensing luer tip (concealed from view in Figure 1 by the cap C). Since the barrel B of the syringe S is full in Figure 1, the plunger P is extended longitudinally. A flap or substrate 10 for a label 12 is provided by two strips of adhesive tape 14, 16, both of which are wrapped around and adhered to respectively opposite sides of the barrel B and adhered to each other in face-to-face relation in extensions 18, 20 of the adhesive tape 14, 16 that extend in diametrically opposite directions from the barrel B. It is preferred, but not necessary, that at least one of the adhesive tapes 14, 16 be transparent so that the graduation marks G that are on most conventional syringes as well as the plunger piston (not shown) in Figure 1) can be seen through the adhesive tape.

In the syringe shown in Figure 1, the label 12 is a printed sheet that has been adhered to the panel extension 20 of the substrate 10. However, the label could also be provided in other ways. For example, but not for limitation, the printed information could be printed directly on one or both of the adhesive tapes 14, 16. Such printing, if placed on a transparent tape 14, 16 would preferably not be enough to mask the graduation marks G. Another option could be to make one of the tapes, such as tape 14 opaque, perhaps with label information on it, but make the other tape 16 transparent so as not to mask or hide the graduation marks G. For another possibility, a sheet label similar to label 12 could be sandwiched between the two adhesive tapes 14, 16.

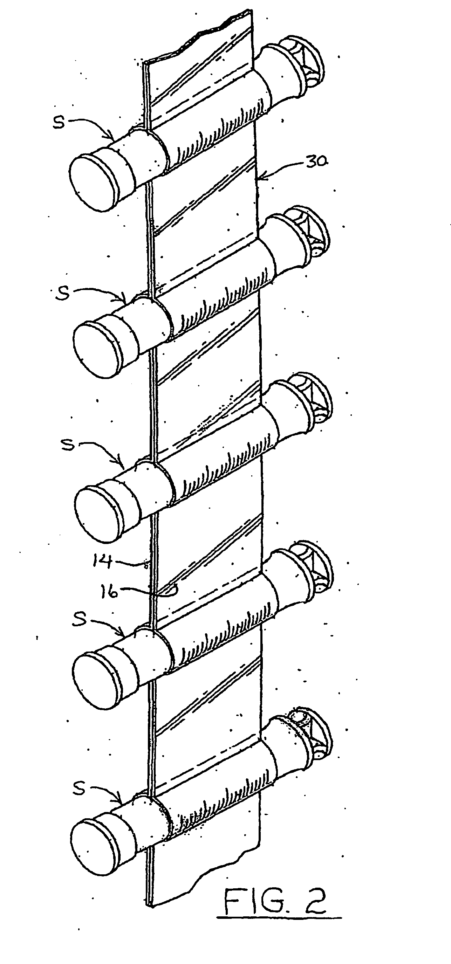

As mentioned above, a significant feature of the described methods and apparatuses is having a plurality of sterile, capped syringes S mounted in spaced apart relation to each other in a band or belt 30, as shown in Figure 2, for handling the syringes S in automated preparation operations. For example, belt 30 may be employed for pulling the syringes S into and preferably at least partially through a labeling and/or filling apparatus and process, as will be described in more detail below. The band or belt 30 can be made with the two elongated adhesive tapes 15, 16 that were described above and which can be cut to separate the syringes S into individual syringes S with the label substrate 10 as shown in Figure 1 and as will be described in more detail below.

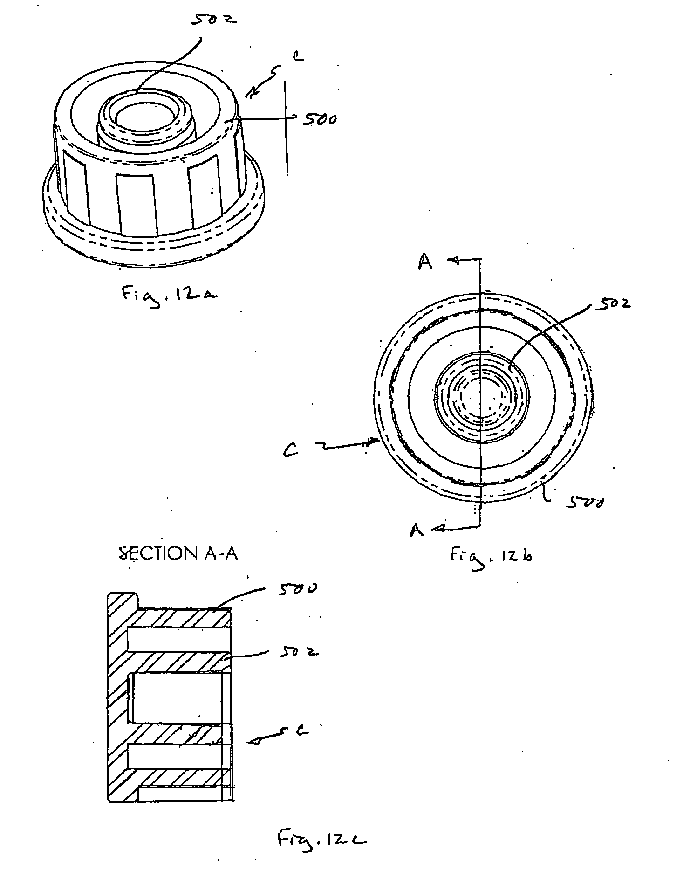

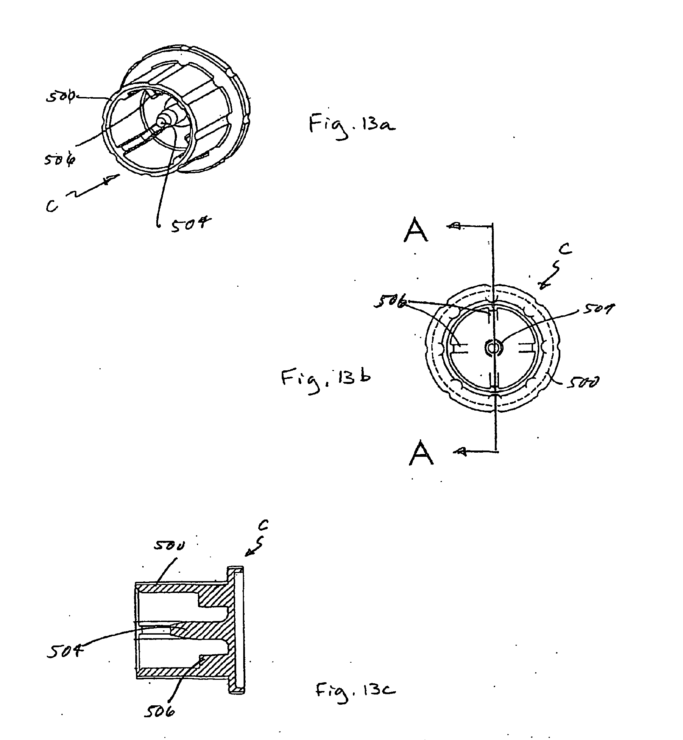

Before proceeding, reference is now made to Figures 12a-12c and Figures 13a-13c which illustrate alternate caps C employable with syringes S of the type shown in Figures 1 and 2. As shown, the caps C each include a cylindrical outer member 500 for matingly engaging the outer flange provided at the dispensing end of the barrel B of the syringe S. In Figure 12a-12c, a cylindrical inner member 502 is also provided for matingly receiving the fluid port provided at the dispensing end of barrel B of syringe S. In the case of the embodiment shown in Figures 13a-13c a central pin-like inner member 504 is provided for mating insertion into the fluid port provided at the dispensing end of the barrel B of syringe S. Of further note, internal locating legs 506 are provided in Figure 13a-13c for retentively engaging the fluid port of barrel B. As may be appreciated, the caps of Figure 12a-12c and Figure 13a-13c both provide for isolation of the contents of syringe S.

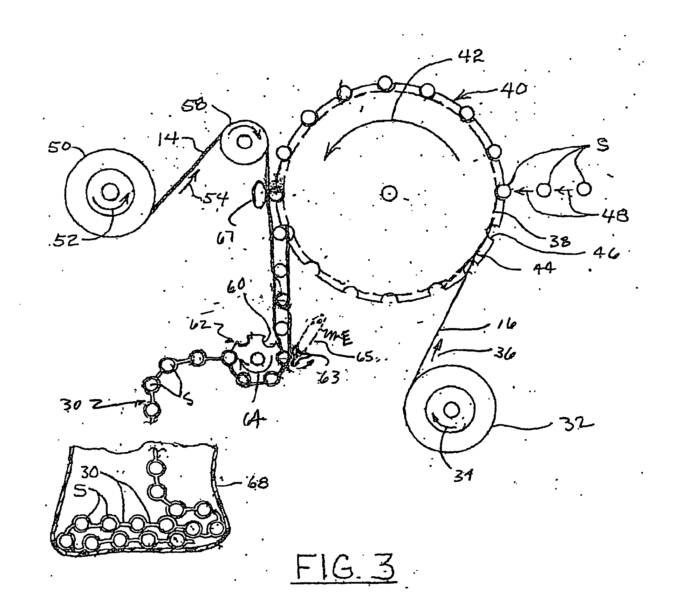

There are many ways by which the plurality of syringes S can be mounted in the band or belt 30 shown in Figure 2, and this disclosure is not limited to any one of such ways of doing so. However, for purposes of example, but not for limitation, one method and apparatus for mounting multiple syringes S into a band or belt 30 is shown in Figure 3. As one tape strip, e.g., tape strip 16, is unwound from a roll 32, as indicated by arrows 34, 36, it is threaded around the periphery 38 of a syringe mounting wheel 40, which rotates as indicated by arrow 42. A pair of rims (only one rim 44 of the pair can be seen in the elevation view of Figure 2) extend radially outward beyond each side of the periphery 38, and each of the rims 44 has a plurality of notches 46 in equal, angularly spaced relation to each other around the periphery 38. As the wheel 40 rotates, preferably capped, empty syringes S are placed serially into the notches 46, as indicated by arrows 48, where they contact the adhesive side of the tape strip 16.

As the wheel 40 rotates, as indicated by the arrow 42, it carries the syringes S in the notches 46 and in contact with the tape strip 16 to a position where the syringes S come into contact with the adhesive side of the other tape strip 14, which is simultaneously being unwound from a roll 50 as indicated by arrows 52, 54, 56. An idler wheel 58 positions the tape strip 14 in relation to the wheel 40 so that it contacts the syringes S mounted in the notches 46. Therefore, the tapes strips 14, 16 get adhered to diametrically opposite sides of the syringes S. In this regard, a contact plate 67 may also be provided to insure engagement between tape strip 14 and syringes.

As the syringes S, which are adhered to tape strips 14, 16 emerge from the wheel 40, they are captured by notches 60 in a press wheel 62 that rotates, as indicated by arrow 64, to press the tape strips 14, 16 to each other between the syringes S. Press wheel 62 may be provided for driven rotation, wherein such driven rotation effects rotation of the tape rolls 32 and 50, as well as rotation of syringe mounting wheel 40 as the tape strips 14, 16 are pulled around press wheel 62 with syringes S secured therebetween. A rotatable pressing block 63 is juxtaposed to the press wheel 62 so that the tape strips 14, 16 run between the press wheel 62 and the rotatable pressing block 63. The pressing block 63 may be configured to present a plurality of semicircular surfaces that are spaced to be in opposing relation to notches 60. Thus, the press wheel 62 and the pressing block 63 cooperate to press and adhere the tape strips 14, 16 tightly together and around the circumference of each syringe S. The pressing block 63 is preferably yieldably biased by a spring-loaded pivot arm 65 or some other bias system to press the pressing block 63 toward the press wheel 62.

After disengaging from press wheel 62, the belt 30 with the syringes S mounted therein are fed as indicated by arrow 66 into a bin or bag 68. Alternatively, the belt 30 with syringes S could be fed directly into a labeling and/or filling apparatus, which will be described below.

In general, the syringes S are positioned in the band or belt 30 in a common orientation, i.e., with luers of all the syringes S on the same side of the band 30. The notches 46 in the wheel 40 are spaced uniformly around the rim 44, so the syringes S in the resulting band 30 are spaced equidistantly apart. The caps C can be placed on the syringes S either before, while, or after the syringes S are mounted in the band 30. The band 30 of syringes S can then be fan folded or rolled and placed in the plastic bag 68, which can be closed and/or sealed to protect sterility. The package or bag 68 of banded syringes 30 can then be sterilized by any of a variety of standard sterilization processes, for example by gamma radiation. The sterilized packages 68 of sterilized, banded syringes S, usually in quantities of about 200 to 1,000 syringes S per package 68, are shipped to users, such as hospitals or other health care institutions, who will label and/or fill and re-cap the syringes S for use within an acceptable time after filling.

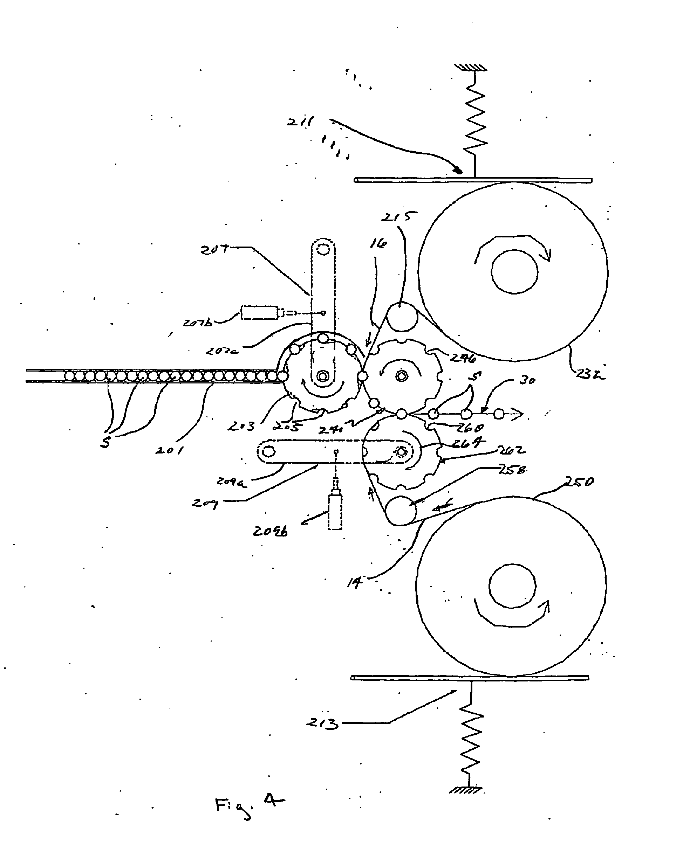

Figure 4 illustrates another method and apparatus for mounting multiple syringes S into a band or belt 30. In this figure a syringe feed-wheel 203 is driven synchronously with tape feed wheels 240 and 262 to form a band 30 of interconnected syringes S. More particularly, tape feed wheels 240 and 262 are driven to pull adhesive tapes 16 and 14 about idler wheels 215 and 258 from tape rolls 232 and 250, respectively. Tensioning devices 211 and 215 are provided to establish a desired amount of tension along tape strips 16 and 14 as they are fed to tape feed wheels 240 and 262, respectively.

As shown by Figure 4, a vibrating track 201 is provided to advance syringes S for sequential loading into notches 205 of the syringe feed wheel 203. In turn, the syringe feed-wheel 203 is located immediately adjacent to the tape feed-wheel 240 so that notches 246 of the tape feed-wheel and notches 205 of the syringe feed-wheel 240 are disposed in opposing relation. As such, it can be seen that tape 16 will be pressed into notches 246 on one side of syringes S to achieve conformal interconnection therewith. Further in this regard, a pneumatic position and tension control device 207 is provided to enhance the interconnection between syringes S and tape 16. Device 207 includes a mount lever arm 207a interconnected to the syringe feed-wheel 203, and a pneumatic cylinder 207b for locating the arm 207a and syringe feed-wheel 203 as appropriate so that syringes S apply a predetermined, desired amount of force against tape 16.

After interconnection of one side of syringes S to adhesive tape 16, Figure 4 provides for the interconnection of adhesive tape 14 to the other side of syringes S. More particularly, tape feed-wheel 262 is driven synchronously with and positioned relative to tape feed-wheel 240 so that notches 260 are in aligned relation with notches 246 to capture syringes S between adhesive tape strips 14 and 16. Concomitantly, tape 14 is pressed about the syringes S to complete band 30.

As further shown in Figure 4, a pneumatic position and tension control device 209 is provided at the tape feed-wheel 262. Device 209 includes a mount lever arm 209a and a pneumatic cylinder 209b for locating the tape feed-wheel 262 as appropriate to establish the desired amount of force applied by syringes S to tape strip 16.

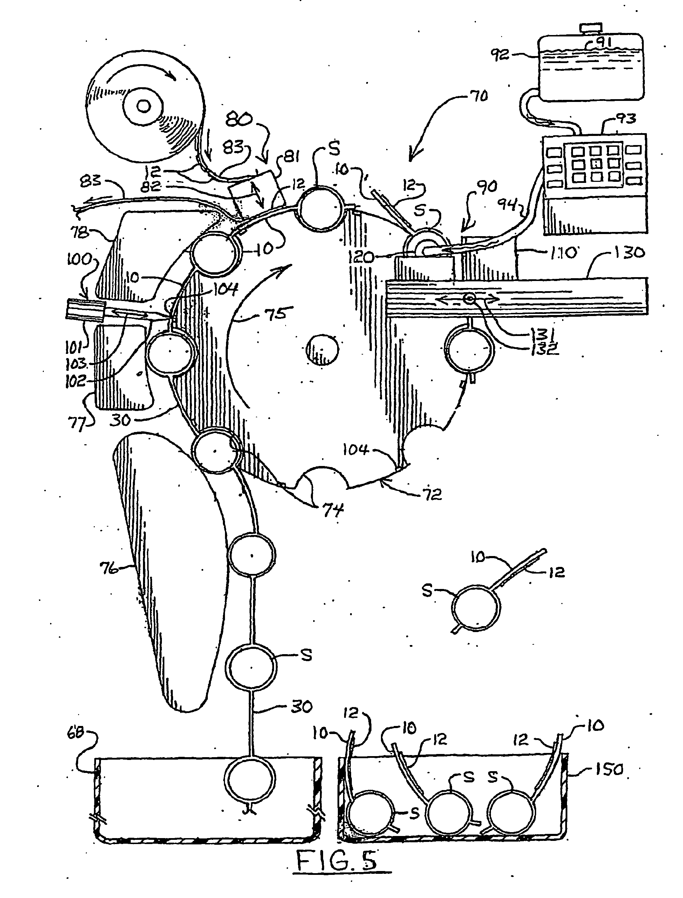

Referring now to the diagrammatic elevation view of the labeling and filling apparatus 70 in Figure 5, a band 30 of syringes S is pulled from the bag 68 by a sprocket wheel or drum 72 and rotated to positions where the band 30 is cut to form the label substrates 10 (see Figure 1), and, if the substrates are not already labeled, to attach labels 12 to the substrates 10, and to remove the caps C, fill the syringes S with the desired medication, and replace the caps C.

In Figure 5, if the bands 30 do not already have labels, the user will prepare a quantity of labels 12 and mount them to feed into a labeling station 80. The labels can be prepared in any suitable manner, for example, using a standard computer label printer, and the quantity of labels 12 prepared can correspond to the number of syringes S to be filled with medication that matches the labels 12. The user also prepares the liquid medication 91 in a container 92, which the user connects to a suitable fluid control system, such as conventional peristaltic pump 93 or other suitable syringe filling, fluid metering, or handling system. The medication will be conveyed via a suitable tube 94 or other conduit to the syringe filling station 90, which will be explained in more detail below. The volume of medication to be pumped into each syringe S can be set and controlled in any of a variety of ways. For example, the pump 93 can be actuated to initiate a fill and deactuated when the syringe has been filled with the desired volume of medication, as will be described in more detail below.

With continuing reference primarily to Figure 5, the sprocket drum 72 has a plurality of notches 74 in equal, angularly-spaced relation to each other around the circumference of the drum 72. The notches 74 are large enough to receive and retain a syringe S, and they are spaced apart from each other the same distance as the spacing between the syringes S in the band 30. Therefore, when at least one of the syringes S in the band 30 is positioned in an appropriate notch 74, rotation of the drum 72, as indicated by arrow 75, will cause the band 30 to pull successive syringes S in the band 30 out of the bag 68 and into the labeling and filling apparatus 70. Suitable guides, for example, guides 76, 77, 78, can be used to hold the syringes S in the notches 74 as the drum 72 rotates and carries the syringes S through the cutting station 100, labeling station 80, and filling station 90.

It is appropriate to mention at this point that the sequential order of cutting, labeling, and filling is not critical, and these operations can be performed in any sequential order or even simultaneously, depending on how one wishes to mount the appropriate equipment, as would be within the capabilities of persons skilled in the art once the principles of the described method and apparatus are understood. However, the convenient sequence of cutting, labeling, and filling will be used for purposes of this description. The drum 72 can be driven to rotate, as indicated by arrow 75, and to stop with syringes S positioned appropriately for the cutting, labeling, and filling operations at the respective stations 100, 80, 90 by any appropriate drive and control system as is well within the capability of persons skilled in the art, such as, for example, with a stepper motor (not shown) connected to appropriate motor control devices (not shown). A control panel (not shown) connected to the stepper motor can be set up for use by an operator to either jog the drum 72 through incremental steps and/or jog the cutting station 100, labeling station 80, or filling station 90 through their respective operations or to initiate continuous automatic operation.

At the cutting station 100, an actuator 101 drives a knife blade 102 as indicated by arrow 103 to cut and sever the band 30 to disconnect the syringes S from each other and to leave the resulting band segments or flaps connected to each syringe S to form individual label substrates 10 for each syringe S. The knife blade 102 is preferably serrated and a slot 104 in the drum in alignment with the knife blade 102 facilitate sure, complete cuts. Any suitable actuator 101 can be used, such as a rotary drive motor, solenoid, or the like. A sheath (not shown) can be provided to cover the blade 102 when it is not cutting. An optical or other sensor (not shown) can be positioned adjacent the drum 72 where the syringes S are first engaged by notches 74 to detect whether any syringes S have missing caps. A signal from the sensor in response to a missing cap could actuate and alarm and/or shut down the apparatus to prevent an uncapped syringe S from being labeled and filled.

For the syringe S that has advanced to the labeling station 80, a labeler device 81, moving as indicated by arrow 82, affixes a label 12 to the substrate 10. The labeler device 81 can be any of a variety of known label apparatus that transfer labels 12 from a strip 83 to an object, or it could be some other device, such as printer apparatus that prints the label directly onto the flap substrate 10, or some combination of such apparatus, as would be within the capabilities of persons skilled in the art once they understand the principles of this disclosed apparatus. An optical sensor (not shown) is used to detect whether a label has been affixed to the substrate 10 at the label station 80. A microprocessor (not shown) can be used to keep count of labels properly affixed and/or activate an alarm and/or shut down the apparatus 70 if a label is not detected on a substrate where a label is supposed to be affixed.

For a syringe S that has advanced to the fill station 90, the cap C (not shown in Figure 5) is removed by a cap handling apparatus 110, then a liquid dispensing apparatus 120 is connected to the luer (not shown in Figure 5) of the syringe S to dispense liquid medication into the syringe S, and the pump 93 (or other suitable liquid metering or control apparatus) is actuated to move the medication 91 from the container 92 into the syringe S. When the syringe S is filled with the desired volume of fluid, as sensed, for example, by a proximity sensor that senses the corresponding desired position of the plunger P (not shown in Figure 4) of the syringe S, the pump 93 (or other suitable liquid metering or control apparatus) is deactuated. Then, the liquid dispensing apparatus 120 is disconnected from the syringe S, and the cap handling apparatus 100 is moved into position to replace the cap C (not shown in Figure 5) onto the luer (not shown in Figure 4) of the syringe S. The cap handling apparatus 110 and the liquid dispensing apparatus 120 are mounted on a cammed shuttle 130, which moves laterally in two axes, as indicated by arrow 131 in the plane of the paper and by arrow 132 perpendicular to the plane of the paper, to accomplish the cap removal, fill, and cap replacement functions described above. While these functions could be performed by myriad other devices and combinations of devices, as would be within the capabilities of persons skilled in the art once they understand the principles of this disclosure, an example cammed shuttle 130, cap handling apparatus 110, and liquid dispensing apparatus 120 shown diagrammatically in Figure 4 will be described in more detail below.

After the syringes S leave the fill station 90, they are allowed to drop individually out of the sprocket drum 72 and, for example, into a basket 150 or other receptacle. At this stage, the syringes S are labeled, filled, and ready for use, as shown in Figure 1.

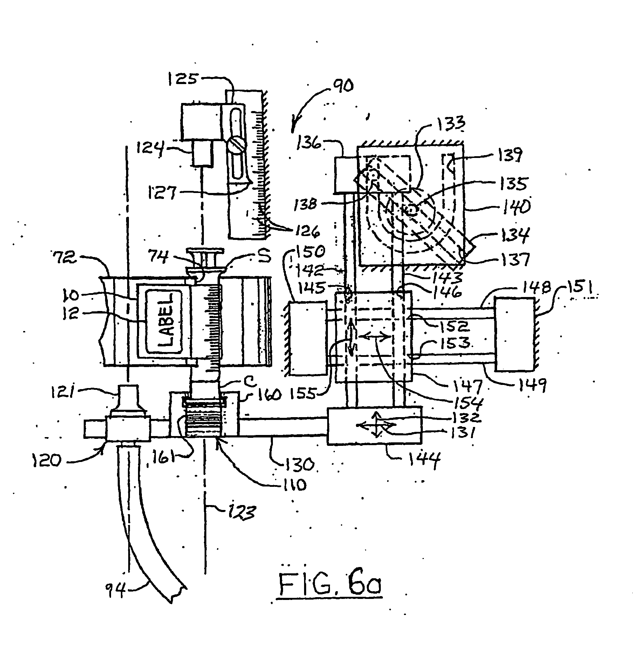

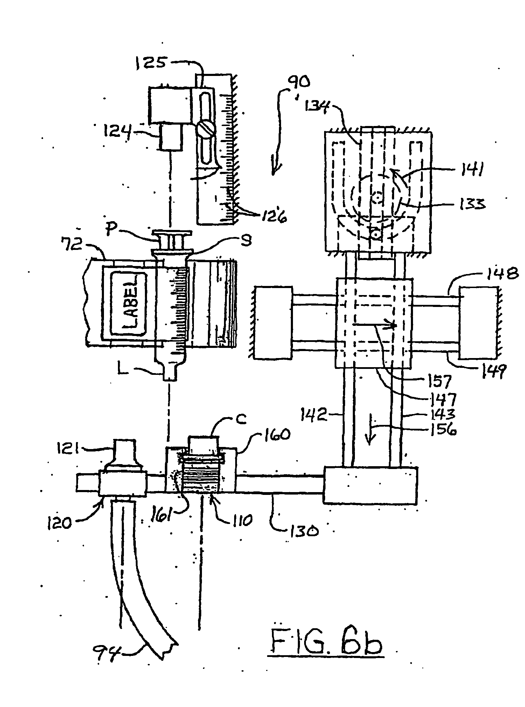

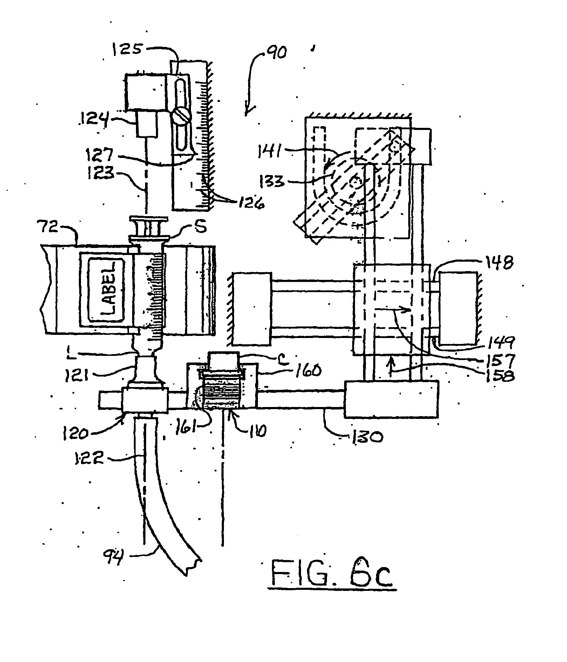

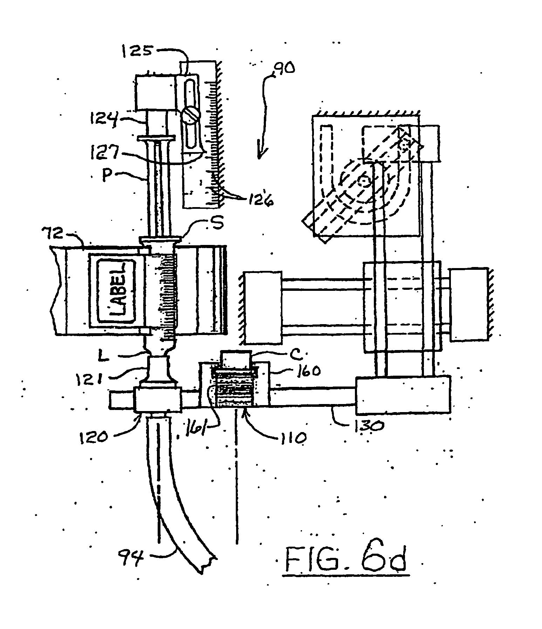

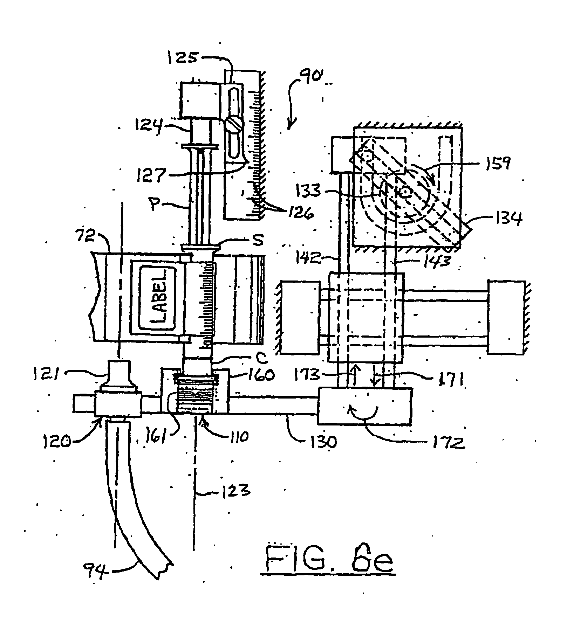

Referring now to Figures 6a, 6b, 6c, 6d, and 6e in combination with Figure 5, the cammed shuttle 130 is driven by a motor, such as a stepper motor 133, which rotates a slotted cam lever or crank arm 134 mounted on the drive shaft 135 of the motor 133. A driver block 136 has a slide pin or a cam roll (concealed from view) extending in one direction into the slotted race groove 137 of the cam lever or crank arm 134 and another cam follow pin or cam roll 138 extending in the opposite direction into a U-shaped cam slot 139 in a stationary cam block 140. Therefore, as the stepper motor 133 rotates, for example as shown by arrow 141 in Figures 6b and 6c, the cam lever 134 causes the cam follower pin or cam roll 138 extending from the driver block 136 to follow the U-shaped path of the cam slot 139, which moves the two slide shafts 142, 143 extending laterally from driver block 136 as well as the connecting block 144 at the distal ends of slide shafts 142, 143 to move simultaneously in the same U-shaped motion pattern. The two slide shafts 142, 143 extend slidably through two holes 145, 146 in a pillow block 147, which is mounted slidably on two support rods 148, 149. The support rods 148, 149 are mounted in two stationary anchor blocks 150, 151 and extend slidably through two holes 152, 153 in pillow block 147, which are perpendicular to, but vertically offset from, holes 145, 146. Thus, as the stepper motor 133 drives the driver block 136 through the U-shaped pattern of cam slot 139, the pillow block 147 slides laterally on support rods 148, 149 as indicated by arrow 154, while the slide shafts 142, 143 slide longitudinally in pillow block 147 as indicated by arrow 155. As a result, the connector block 144 and cammed shuttle 130 also move both laterally and longitudinally as indicated by arrows 131, 132 in the same U-shape pattern as the U-shaped cam slot 139 to remove the cap C from the syringe S, connect the syringe S to a nozzle 121 in the liquid dispensing apparatus 120 to fill the syringe S, disconnect the nozzle 121, and replace the cap C, as will be described in more detail below. Suitable bushing or bearings can be used to enhance the sliding movement of the shafts 142, 143 and support rods 148, 149 in the pillow block 147.

Referring now to Figure 6a in combination with Figure 4, the drum 72 has moved a syringe S to the filling station 90, where it stops for the cap removal, fill, and cap replacement operation. The syringe S is shown in Figure 6a positioned in a notch 74 with a label 12 affixed to the substrate 10. As the drum 72 moved the syringe S to the position shown in Figure 6a, the cap C was moved into a set of jaws 160, which is aligned longitudinally with the syringe S when the slotted cam lever 134 is stopped in the position shown in Figure 6a and the drum 72 stops the syringe S in the filling station 90. A cap gripper 161, such as resilient spring steel, presses against the cap C in jaws 160 to capture and retain the cap C in the jaws 160. Again, optical sensors (not shown) or other suitable sensors and/or control devices or methods can be used to stop the drum 72 when the syringe S is positioned with the cap C captured in the jaws 160 as would be understood by persons skilled in the art once they understand the principles of this disclosure. Then, the motor 133 is actuated to rotate the slotted cam lever 134 as indicated by arrow 141 in Figure 6b, which extends the slide shafts 142, 143, as indicated by arrow 156, as the pillow block 147 slides to the right on support rods 148, 149, as indicated by arrow 157. As a result, the cammed shuttle 130 moves the jaws 160 with the cap C away from the syringe S, thereby removing the cap C from the syringe S and leaving the luer L of the syringe S exposed and open, as shown in Figure 6b. Again, the gripper 161 described above retains the cap C in the jaws 160 when the cap C is removed from the luer L.

Continued rotation of the cam lever 134 as indicated by the arrow 141 in Figure 5c translates the pillow block 147 still farther to the right on support rods 148, 149, as indicated by arrow 157 in Figure 6c, until the longitudinal axis 122 of the fill connector or nozzle 121 aligns with the longitudinal axis 123 of syringe S, then retracts the slide shafts 142, 143, as indicated by arrow 158, to position the nozzle 121 on luer L of the syringe S. At that position of the cammed shuttle 130, the motor 133 is deactuated, so the nozzle 121 stays on the luer L while the pump 93 (Figure 5) is actuated to pump liquid medication 91 from the container 92 to fill the syringe S. The fill connector or nozzle 121 is preferably mounted on the cammed shuttle 130 by a spring-loaded slide (not shown) or similar yieldable, resilient mounting to apply an appropriate, uniform force to the nozzle 121 as it is being forced by the cammed shuttle 130 onto the luer L of the syringe S. This motion to remove the cap C and place the fill connector or nozzle 121 on the syringe S can be accomplished in approximately 250 milliseconds with this mechanism. The U-shaped cam slot 139 provides a straight, longitudinal pull of the cap C in alignment with the longitudinal axis 123 of the syringe S and a corresponding straight, longitudinal push to attach the nozzle 121 to the luer L.

As best seen in Figure 6d, the plunger P of the syringe S is pushed outwardly by the liquid medication that is pumped into the syringe S. When the syringe S has been filled with the desired volume of liquid medication, the flow of liquid medication into the syringe S is stopped. The flow can be measured and stopped in a variety of ways, such as flow meters, valves, known pump displacement, and the like, as would be within the knowledge and capabilities of persons skilled in the art once they understand the principles of this disclosure. However, a particularly novel and innovative way of controlling the fill volume is to use a sensor 124 to detect when the plunger P has been pushed out to a predetermined extent that corresponds to the fill volume desired, as illustrated in Figure 6d a myriad of sensors could be used for this function, such as a capacitive proximity sensor, optical sensor, microswitch, and the like. Upon sensing the desired extension of the plunger P, a signal from the sensor 124 can be used to shut off the flow of liquid medication into the syringe S. A suitable signal control circuit, for example, a microprocessor and/or relay, (not shown) to shut off the pump 93 or to close some control valve (not shown) is well within the capabilities of persons skilled in the art once they understand the principles of this disclosure. As shown in Figure 6d, the sensor 124 can be mounted on an adjustable base 125 with a scale 126 and pointer 127 to correlate adjustable physical position of the sensor with the desired fill volume.

When the desired fill volume has been reached and detected, as explained above, a signal from the sensor 124 is used to deactuate the pump 93. A preferred, albeit not essential, pump 93 is a peristaltic pump, such as, for example, a model 099 Repeater Pump, manufactured by Baxa Corporation, of Englewood, Colorado, which can be reversed momentarily to take the fluid pressure off the tubing 94 and syringe S to minimize, if not prevent, dripping of the liquid medication when the nozzle 121 is detached from the luer L. Then, the motor 133 is actuated to rotate the cam lever 132 in the opposite direction, as indicated by the arrow 159 in Figure 6e, to detach the nozzle 121 from the luer L of the syringe S and move the jaws 60 and cap C back into longitudinal alignment with the axis 123 of the syringe S for replacing the cap C on the syringe S. Specifically, as the cam lever 134 rotates, as shown by arrow 159, the cammed shuttle 130 moves back through the U-shaped pattern defined by the U-shaped cam slot 139. First, the slide shafts 142, 143 are extended as indicated by arrow 171 to detach the nozzle 121 from the luer L of syringe S. Then the cammed shuttle is moved in an arc as indicated by arrow 172 to align the cap C in jaws 160 with the longitudinal axis 123 of the syringe S. Finally, the slide shafts 142, 143 are retracted again, as indicated by arrow 173, to push the cap C back onto the syringe S. The cap handling apparatus 110 can be mounted by a spring-loaded slide (not shown) or some other yieldable, resilient structure, if desired, to ensure a uniform pressure application to the cap C as it is being pushed by the cammed shuttle 130 back onto the syringe S.

At this position, shown in Figure 6e, the fill is completed, and the drum 72 can be rotated again to move the cap C out of the jaws 160 and to move the next syringe S in the sequence into the jaws 160 for a repeat of the cap removal, fill, and cap replacement sequence described above on the next syringe S in the drum 72. At the next position after the filling station 90, a sensor (not shown), such as an optical sensor, is used to determine if the cap C is placed correctly back on the syringe S. If it is not placed correctly, the apparatus is stopped and/or an alarm is sounded in response to a signal from the sensor indicating that the cap C is not replaced. After that cap-check position, the drum moves the syringe to a point where hold down or guide tracks end, thereby freeing the syringe S to drop out of the drum 72 and into a chute (not shown) that guides the labeled, filled, and recapped syringe S into the holding basket 150.

The control system (not shown) can utilize signals from the sensors to record number of syringes S filled, program the number of doses desired and automatically stop when that number of syringes S are filled, record the number of doses actually pumped, record the number of doses or syringes in the basket 150 and keep track of rejected labels or syringes. Other functions can also be provided.

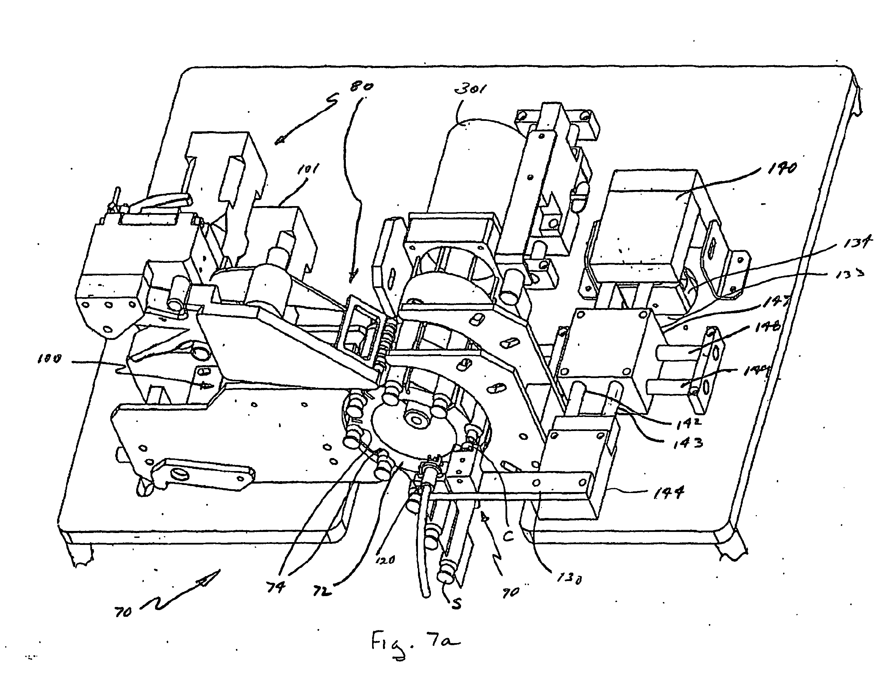

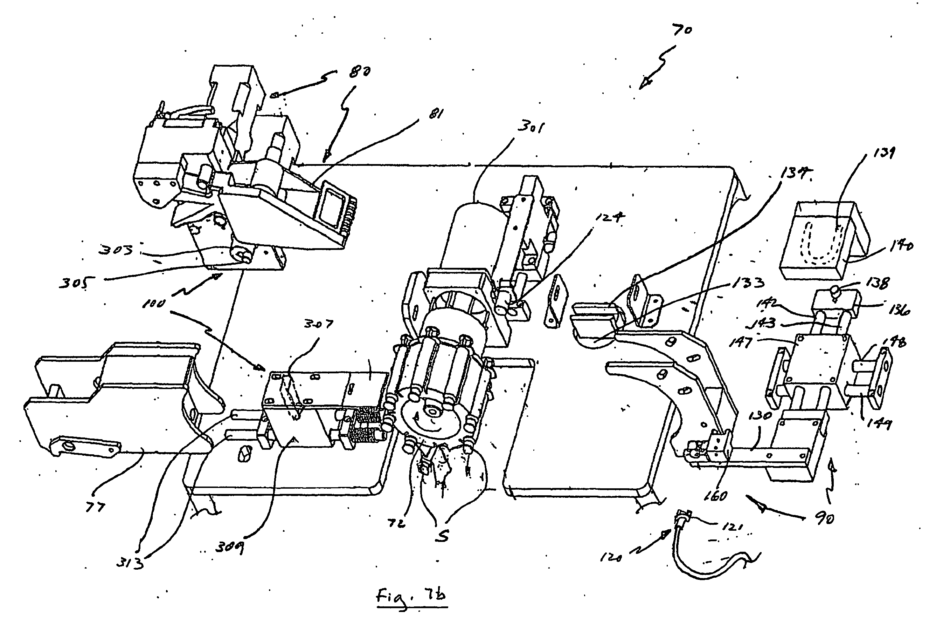

Referring now to Figures 7a and 7b, the labeling and filling apparatus of Figure 5 and Figure 6a-6e is further illustrated in a production implementation. Of note, the labeling and filling apparatus 70 is shown in a compact table top arrangement that may be readily positioned in a sterile environment, e.g. within a sterile area having an appropriate exhaust hood. As will be recognized, the apparatus 70 includes a cutting station 100, labeling station 80 and filling station 90.

The drum 72 may be driven in a clockwise direction by a step motor 301, wherein syringes S are positioned into the notches 74 for sequential feeding to the work stations 80, 90 and 100. At cutting station 100, an actuator 101 in the form of a stepper motor may be utilized. In particular, the actuator 101 may be controlled to turn a crank 303 having a cam follower 305 that is located in a slot 307 on a mount block 309 for cutting blade 102. The block 309 is supported on rails 311, wherein driven rotation of the crank 303 effects linear travel of the cutting blade 102 towards and away from the drum 72 and a belt 30 with syringes S carried thereby. The operation of actuator 101 may be timed in relation to the stepped movement of drum 72 so that belt 30 is cut into belt segments 10 of a consistent width by cutting blade 102.

At labeling station 80, the labeling device 81 may include a stepper motor 315 to which a shaft 317 is interconnected for driven eccentric motion. That is, upon actuation stepper motor 315 may drive shaft 317 through an arc from a first position to a second position. By way of example, the first position may be as illustrated in

Figures 7a and 7b, wherein the labeling device 81 is located in a down position for label placement. Upon eccentric motion of the shaft 317 to a second position, shaft 317 will engage the labeling device 81 causing the cantilevered end thereof to cock upwards about a stationary shaft 319. As may be appreciated, the operation of stepper motor 315 is timed in relation to the stepped movement of drum 72 to affect label placement on the belt segments 10 between adjacent syringes S.

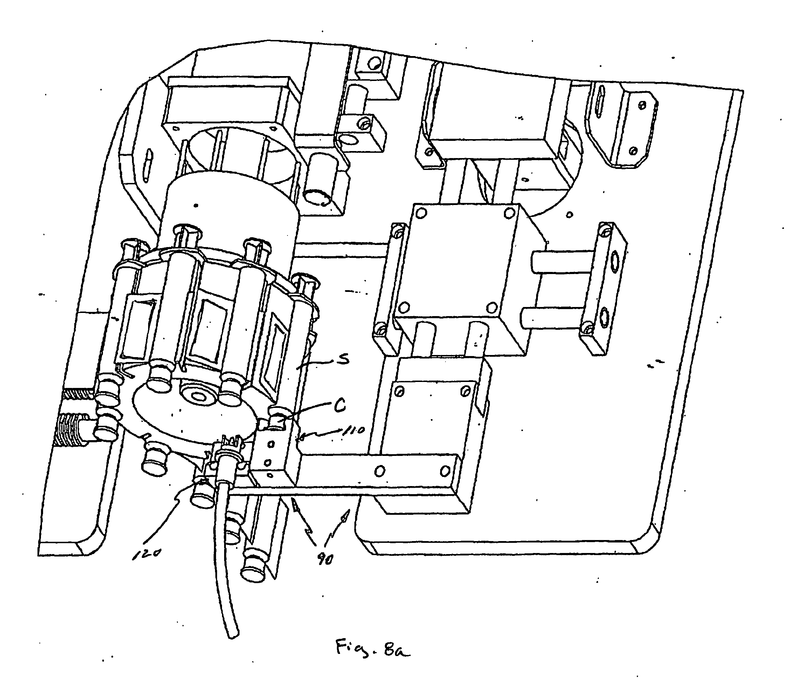

Referring now to Figures 8a-8d, operation of the filling station 80 shown in Figures 7a and 7b will be further described. In Figure 8a a syringe S has advanced to the filling station 90 with a cap C inserted into cap handling apparatus 110. As illustrated, syringe S has an interconnected belt segment on flap 10 with a label 12 adhered thereto.

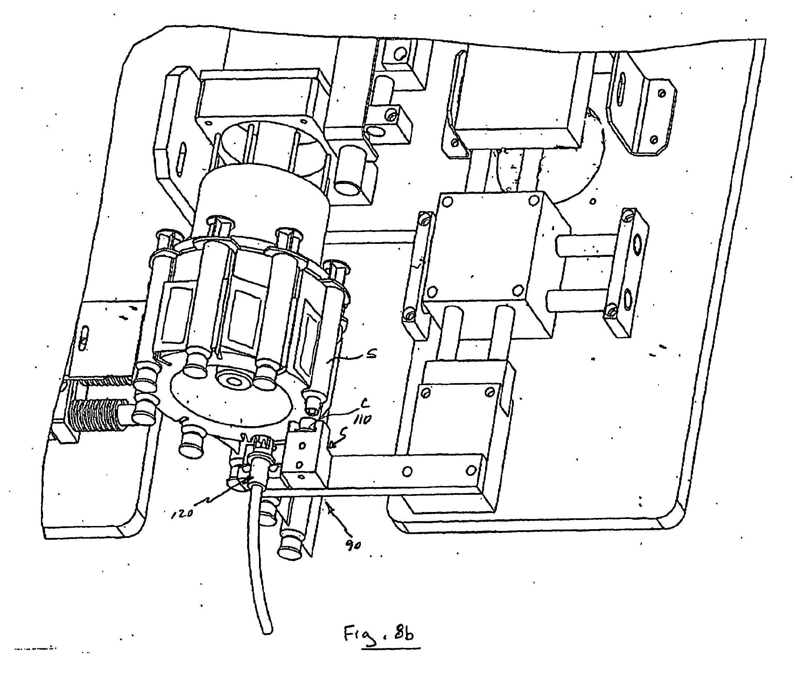

As next shown in Figure 8b, it can be seen that filling station 90 has retracted away from drum 72 so as to remove cap C from the dispensing end of the syringe S. As previously noted, such retraction is achieved by activating stepper motor 133 to rotate cam lever 134, thereby causing driver block 136, slide shafts 142, 143, connecting block 144 and shuttle 130 to move along a first straight leg portion of U-shaped motion pattern.

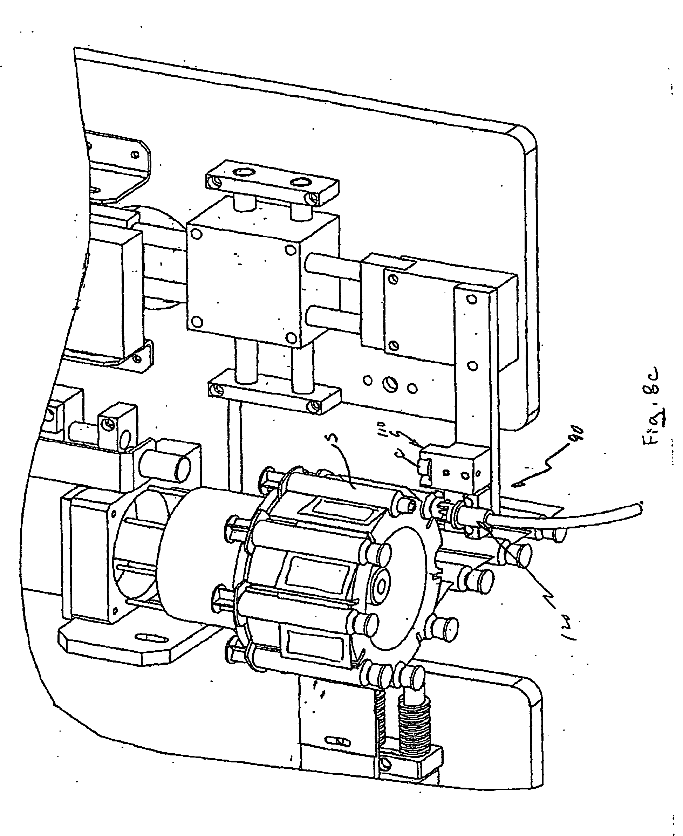

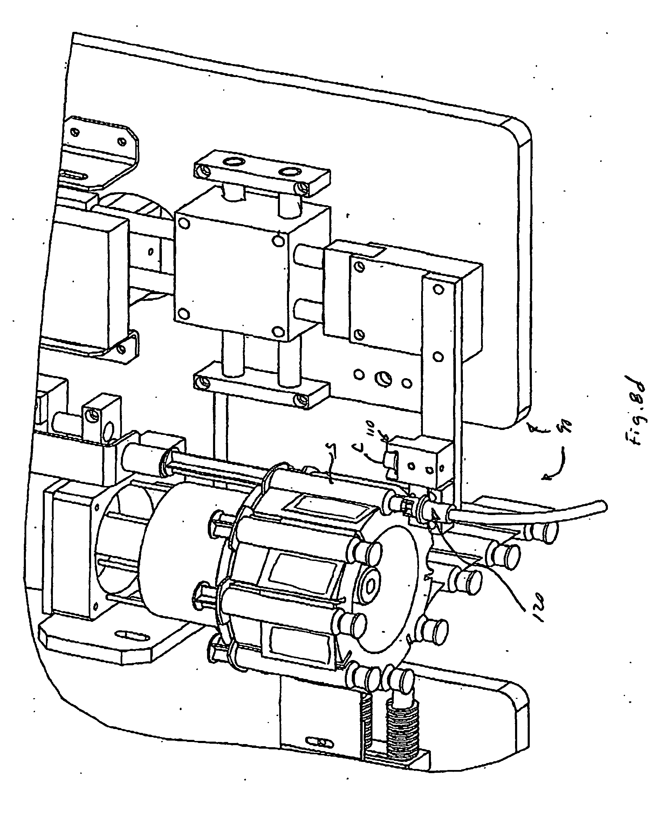

In the later regard, Figure 8c shows the filling station 90 immediately after cam lever 134 has moved through the curved portion of the U-shaped motion pattern. In this position it can be seen that the nozzle 121 of the liquid dispensing apparatus 120 is aligned with the dispensing end of the syringe S. As such, and as seen in Figure 8d, further movement of the filling station 90 along the second straight leg portion of the U-shaped motion pattern causes the liquid dispensing apparatus 120 to linearly advance towards syringe S, wherein the nozzle 121 engages and fluidly interconnects with the dispensing end of the syringe S. Upon reaching the Figure 8d position, filling station 90 may be controlled so that fluid is injected through nozzle 121 into the syringe S. As further shown in Figure 8d, fluid has filled the syringe S to displace the plunger P into contact with the sensor 124. At this point, a sensor signal is transmitted to terminate the filling of syringe S. Thereafter, stepper motor 133 may again rotate cam lever 134 through the U-shaped motion pattern to reposition cap C back onto the dispensing end of the syringe S.

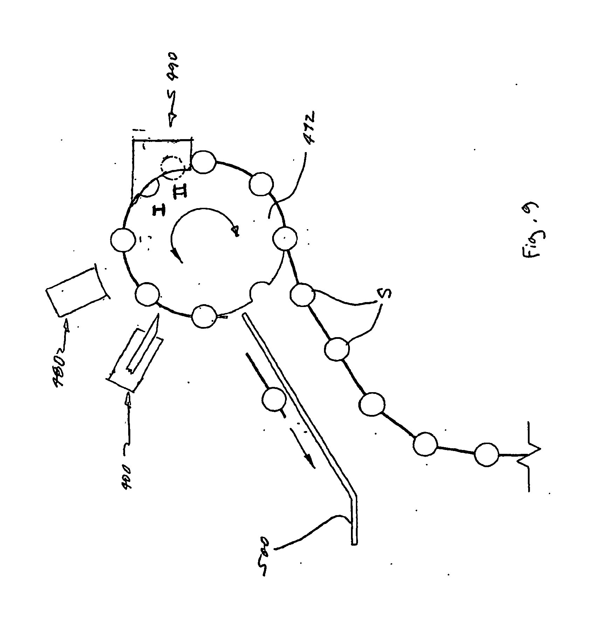

As noted above, the filling and labeling apparatus 70 is only one of methods and apparatuses disclosed herein. Numerous other embodiments will be apparent to those skilled in the art. By way of example, reference is now made to Figures 9, 10 and 11a-11f, which illustrate an alternate method and apparatus.

In this disclosure a drum 472 is driven in a counter-clock wise direction, wherein a band 430 of syringes S pulled in series into the notches 474 for preparation operations. In the later regard, the band 430 is suspended from the drum 472 to facilitate aligned, side-by-side positioning of the syringes S in notches 474. As schematically shown in Figure 9, the syringes S are sequentially advanced through filling station 490, labeling station 480 and cutting station 400. Thereafter, the separated syringes S may be directed into a container (not shown) via a chute 500. The operation of labeling station 480 and cutting station 400 may be analogous to the operations of the labeling station 80 and cutting station 100 described above in relation to Figure 5 and Figures 6a-6b. In contrast to these figures, however, the method and apparatus shown in Figures 9, 10 and 11a-11h may implement a different approach at filling station 490.

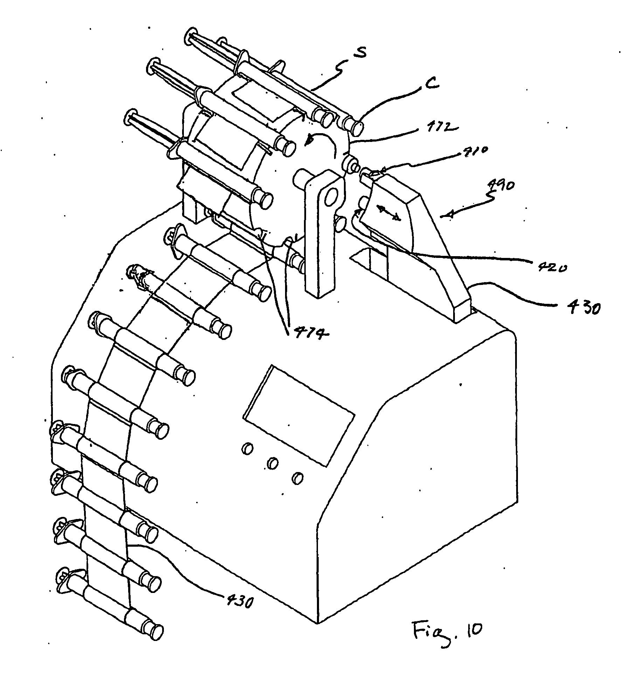

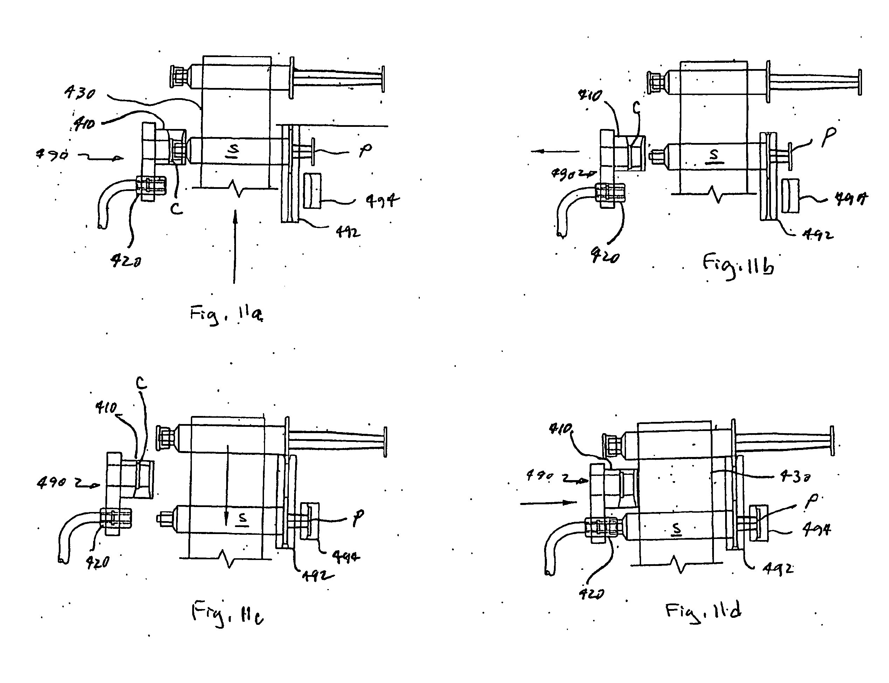

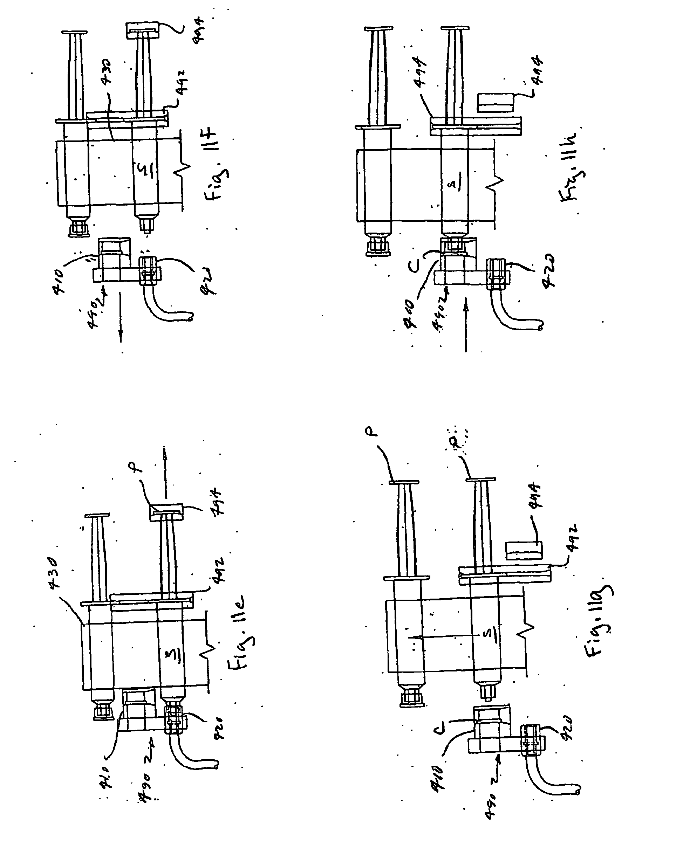

In the modified operation shown in Figure 9, a syringe is first positioned at location I for cap removal, then located at a second position II for filling, followed by location back at work location I for cap replacement. To facilitate an understanding of such approach, the labeling station 480 and cutting station 400 are not presented in Figure 10. As best shown by Figure 10, filling station 490 includes a cap handling apparatus 410 and liquid dispensing apparatus 420. As will be appreciated, liquid dispensing apparatus 420 is interconnectable to a reservoir (not shown) containing a fluid for filling syringes S. Of note, both the cap handling apparatus 41 and liquid dispensing apparatus 420 are mounted on a common support member 430. Support member 430 may be interconnected to a stepper motor (not shown) acutatable to affect linear travel of the cap handling apparatus 410 and liquid dispensing apparatus 420 towards and away from the drum 472. Such linear travel, together with the rotation of drum 472 are the only required motions for cap removal, filling and cap replacement. Such operations will now be further described with reference to Figures 11a-11h.

Figures 11a-11h are flat, diagrammatic views of filling station 490 from a rearward perspective relative to the isometric front view shown in Figure 10. Before proceeding, it should be noted that the filling station 490 shown in Figures 11a-11h further includes a syringe flange retention track 492 and a plunger flange retention member 494. As will be further described, the plunger flange retention number 494 is selectively retractable relative to retention track 492 so that fluid may be drawn from liquid dispensing apparatus 420 to fill syringes S. In this regard, liquid dispensing apparatus 420 may include a valve to control the passage/stoppage of fluid therethrough. By way of example, such valve may comprise an actuatable roller..

With particular reference to Figure 11a, a syringe S is shown in the first location I shown in Figure 9 wherein Cap C has been inserted in the cap handling apparatus 410 for retention thereby. Concomitantly, a flange on syringe S has been inserted and advanced within the retention track 492. Next, and as shown in Figure 11b, cap handling apparatus 410 has been retracted from the syringe S with cap C retained thereby. As will be appreciated, such retraction may be affected via linear driven travel of the support member 430 shown in Figure 10.

Figure 11c shows the syringe S moved to the location II shown in Figure 9. More particularly, drum 472 may be rotated clockwise to affect such positioning, wherein the liquid dispensing apparatus 420 is aligned with the dispensing end of the syringe S. Then, liquid dispensing apparatus 420 may be advanced into engagement with the dispensing end of syringe S as shown in Figure 11d. Again, such linear travel may be affected via movement of support member 430. Of note, both Figures 11c and 11d show the plunger P being positioned in the retention member 494.

In this regard, and referring now to Figure 11e, retention member 494 may be provided for driven retraction away from syringe S (e.g. via an unshown stepper motor), with the valve of liquid dispensing apparatus 420 opened so as to draw fluid through liquid dispensing apparatus 420 into the syringe S. As may be appreciated, the amount, or length, of retraction of retention member 494 may be precisely controlled to achieve a preset filling volume. When the desired volume has been reached, the valve of liquid dispensing apparatus 420 may be closed. Where an actuatable roller is utilized, the roller may be positioned to pinch off a fluid conduit to back up the fluid a desired amount, thereby bringing the fluid pressure slightly below atmospheric pressure. After filling, the liquid dispensing apparatus 420 may be withdrawn from the dispensing end of the syringe S as shown in Figure 11f. Again, such linear travel may be affected by controlled retraction of the support member 430.

Thereafter, syringe S may return to location I via counter-clockwise rotation of drum 472, as shown in Figure 11g. Finally, cap C may be replaced onto the dispensing end of the syringe S via advancement of the cap handling apparatus 410 on support member 430. The syringe S may then be advanced for further operations at the labeling station 480 and cutting station 400 shown in Figure 9.

The foregoing description is considered as illustrative only of the principles of the invention and the methods and apparatuses disclosed herein. Furthermore, since numerous modifications and changes will readily occur to those skilled in the art, it is not desired limit the invention to the exact construction and process shown and described above. Accordingly, resort may be made to all suitable modifications and equivalents that fall within a scope of the invention as defined by the claims which follow. The words "comprise," "comprises," "comprising," "include," "including," and "includes" when used in this specification are intended to specify the presence of stated features, integers, components, or steps, but they do not preclude the presence or addition of one or more other features, integers, components, steps, or groups thereof.

## Claims

1. A method for filling a plurality of syringe bodies (S), wherein for each syringe body (S) of the plurality of syringe bodies (S) the method comprises:
holding the syringe body (S) in at least one holder (74);
removing a cap (C) from a dispensing end of the syringe body (S) during said holding step;
filling the syringe body (S) at the dispensing end thereof after said removing step and during said holding step, said filling step including:
interconnecting a liquid dispensing apparatus (420) with the dispensing end of the syringe body (S) in an automated manner; and
flowing fluid into the syringe body (S) from the liquid dispensing apparatus (420), through the dispensing end of the syringe body (S), by drawing the fluid into the syringe body (S) by retraction of a plunger (P) relative to the syringe body (S); and,
replacing a cap (C) removed during the removing step from one of said plurality of syringe bodies (S) on the dispensing end of the syringe body (S) after said filling step and during said holding step.

2. A method as recited in claim 1, wherein for each syringe body (S) the method further comprises:
placing said cap (C) on said dispensing end of the syringe body (S) prior to said holding step; and
sterilizing the syringe body (S) after said placing step and prior to said holding step.

3. A method as recited in claim 2, wherein said placing and sterilizing steps are completed at a first location and said holding, removing, filling and replacing steps are completed at a second location remote from said first location, and wherein for each syringe body (S) the method further comprises:
packaging said syringe body (S) in a container (68) at said first location after said placing step and prior to said holding step; and
unpackaging said syringe body (S) from said container (68) at said second location prior to said holding step.

4. A method as recited in any of claims 1-3, wherein the method further comprises:
positioning said plurality of syringe bodies (S) in a predetermined orientation prior to said holding step; and
interconnecting said plurality of positioned syringe bodies (S) in said predetermined orientation prior to said holding step.

5. A method as recited in claim 4, wherein the method further comprises locating said plurality of interconnected syringe bodies (S) in said predetermined orientation in a plurality of said at least one holders (74) for performing at least one of said holding step, said removing step, said filling step, and said replacing step.

6. A method as recited in any of claims 1-3, wherein the method further comprises locating said plurality of syringe bodies (S) in a plurality of said at least one holders (74) for performing at least one of said holding step, said removing step, said filling step, and said replacing step.

7. A method as recited in any of claims 5-6, further comprising moving said plurality of said at least one holders (74) along a predetermined path during said performing of at least one of said holding step, said removing step, said filling step, and said replacing step.

8. A method as recited in claim 7, wherein said plurality of said at least one holders (74) are located on a support member (72), and wherein said moving step comprises rotating said support member (72).

9. A method as recited in claim 7, wherein a plurality of work locations are located along said predetermined path, and wherein the method further comprises disposing said plurality of syringe bodies (S) in series at said plurality of work locations to complete said performing of at least one of said holding step, said removing step, said filling step, and said replacing step.

10. A method as recited in any of claims 1-9, wherein each of said plurality of syringe bodies (S) comprises a barrel (B) and a plunger (P) slidably disposed in one end of the barrel (B), and wherein cap (C) covers and protects the dispensing end of the syringe body (S) prior to the removing step to maintain a clean internal volume in syringe body (S).

11. A method as recited in any of claims 1-10, wherein for each syringe body (S) the removing step includes:
retainably engaging said cap (C) in a retainer (160); and
moving at least one of said retainer (160) and said at least one holder (74) in an automated manner to affect relative movement between the cap (C) and the dispensing end of the syringe body (S); and
wherein for each syringe body (S) the replacing step comprises:
retainably engaging said one of said caps (C) in a retainer (160); and
moving said holder (74) along a predetermined path to insert said one of said caps (C) into the syringe body (S).

12. A method as recited in any of claims 1-11, wherein said liquid dispensing apparatus (420) includes a valve, and wherein for each syringe body (S) the filling step comprises:
retracting a plunger flange retention member (494) away from the syringe body (S) with the valve open; and,
closing the valve after the retracting step.

13. A method as recited in any of claims 1-12, wherein for each syringe body (S) said removing, filling and replacing steps are completed at a first location of the syringe body (S).

14. A method as recited in any of claims 1-13, wherein for each syringe body (S) the method further comprises sensing the position of a plunger end thereof to terminate said filling step.

15. A method as recited in any of claims 1-14, wherein for each syringe body (S) the cap (C) removed in the removing step is the same as the cap (C) replaced in said replacing step.

16. A method as recited in any of claims 1-15, wherein for each syringe body (S) the method further comprises holding the cap (C) removed from the syringe body (S) until replaced on one of the plurality of syringe bodies (S).

## Patentansprüche

1. Verfahren zum Befüllen einer Vielzahl von Spritzenkörpern (S), worin das Verfahren für jeden Spritzenkörper (S) der Vielzahl von Spritzenkörpern (S) Folgendes umfasst:
Halten des Spritzenkörpers (S) in zumindest einem Halter (74);
Entfernen einer Kappe (C) von einem Abgabeende des Spritzenkörpers (S) während des Halteschritts;
Befüllen des Spritzenkörpers (S) an seinem Abgabeende nach dem Entfernungsschritt und während des Halteschritts, wobei der Befüllungsschritt Folgendes umfasst:
Verbinden eines Flüssigkeitsabgabegeräts (420) mit dem Abgabeende des Spritzenkörpers (S) auf automatische Weise; und
Durchleiten von Flüssigkeit in den Spritzenkörper (S) aus dem Flüssigkeitsabgabegerät (420) durch das Abgabeende des Spritzenkörpers (S) durch Aufziehen der Flüssigkeit in den Spritzenkörper (S) durch Zurückziehen eines Kolbens (P) relativ zu dem Spritzenkörper (S); und
Wiederaufsetzen einer Kappe (C), die während des Entfernungsschritts von einem der Vielzahl von Spritzenkörpern (S) entfernt wurde, auf das Abgabeende des Spritzenkörpers (S) nach dem Befüllungsschritt und während des Halteschritts.

2. Verfahren nach Anspruch 1, worin das Verfahren für jeden Spritzenkörper (S) ferner Folgendes umfasst:
Aufsetzen der Kappe (C) auf das Abgabeende des Spritzenkörpers (S) vor dem Halteschritt; und
Sterilisieren des Spritzenkörpers (S) nach dem Aufsetzschritt und vor dem Halteschritt.

3. Verfahren nach Anspruch 2, worin die Aufsetz- und Sterilisationsschritte an einem ersten Ort durchgeführt werden und die Halte-, Entfernungs-, Befüllungs- und Wiederaufsetzschritte an einem zweiten, von dem ersten Ort entfernten Ort durchgeführt werden, und worin das Verfahren für jeden Spritzenkörper (S) ferner Folgendes umfasst:
Verpacken des Spritzenkörpers (S) in einem Behälter (68) an dem ersten Ort nach dem Aufsetzschritt und dem Halteschritt; und
Auspacken des Spritzenkörpers (S) aus dem Behälter (68) an dem zweiten Ort vor dem Halteschritt.

4. Verfahren nach einem der Ansprüche 1-3, worin das Verfahren ferner Folgendes umfasst:
Positionierung der Vielzahl von Spritzenkörpern (S) in einer vorbestimmten Orientierung vor dem Halteschritt;
und
Verbinden der Vielzahl von positionierten Spritzenkörpern (S) in der vorbestimmten Orientierung vor dem Halteschritt.

5. Verfahren nach Anspruch 4, worin das Verfahren ferner die Anordnung der Vielzahl von miteinander verbundenen Spritzenkörpern (S) in der vorbestimmten Orientierung in einer Vielzahl der zumindest einen Halter (74) umfasst, um zumindest den Halteschritt, den Entfernungsschritt, den Befüllungsschritt und/oder den Wiederaufsetzschritt durchzuführen.

6. Verfahren nach einem der Ansprüche 1-3, worin das Verfahren ferner die Anordnung der Vielzahl von Spritzenkörpers (S) in einer Vielzahl der zumindest einen Halter (74) umfasst, um zumindest den Halteschritt, den Entfernungsschritt, den Befüllungsschritt und/oder den Wiederaufsetzschritt durchzuführen.

7. Verfahren nach einem der Ansprüche 5-6, ferner umfassend Bewegen der Vielzahl der zumindest einen Halter (74) entlang eines vorbestimmten Wegs während der Durchführung zumindest des Halteschritts, des Entfernungsschritts, des Befüllungsschritts und/oder des Wiederaufsetzschritts.

8. Verfahren nach Anspruch 7, worin die Vielzahl der zumindest einen Halter (74) auf einem Trägerelement (72) angeordnet sind und worin der Bewegungsschritt Drehen des Trägerelements (72) umfasst.

9. Verfahren nach Anspruch 7, worin eine Vielzahl von Arbeitsstellen entlang des vorbestimmten Wegs angeordnet sind und worin das Verfahren ferner die Anordnung der Vielzahl von Spritzenkörpern (S) in Reihe an der Vielzahl von Arbeitsstellen umfasst, um die Durchführung zumindest des Halteschritts, des Entfernungsschritts, des Befüllungsschritts und/oder des Wiederaufsetzschritts zu vollenden.

10. Verfahren nach einem der Ansprüche 1-9, worin die Vielzahl von Spritzenkörpern (S) einen Zylinder (B) und einen Kolben (P) umfasst, der gleitend in einem Ende des Zylinders (B) angeordnet ist, und worin die Kappe (C) das Abgabeende des Spritzenkörpers (S) vor dem Entfernungsschritt abdeckt, um ein sauberes Innenvolumen im Spritzenkörper (S) aufrechtzuerhalten.

11. Verfahren nach einem der Ansprüche 1-10, worin der Entfernungsschritt für jeden Spritzenkörper (S) Folgendes umfasst:
festes Eingreifen der Kappe (C) in einer Halterung (160); und
Bewegen zumindest einer der Halterungen (160) und des zumindest einem Halters (74) auf automatische Weise zur Bewirkung einer Relativbewegung zwischen der Kappe (C) und dem Abgabeende des Spritzenkörpers (S); und
Worin der Wiederaufsetzschritt für jeden Spritzenkörper (S) Folgendes umfasst:
festes Eingreifen einer der Kappen (C) in eine Halterung (160); und
Bewegen des Halters (74) entlang eines vorbestimmten Wegs zum Einsetzen einer der Kappen (C) in den Spritzenkörper (S).

12. Verfahren nach einem der Ansprüche 1-11, worin das Flüssigkeitsabgabegerät (420) ein Ventil aufweist und worin der Befüllungsschritt für jeden Spritzenkörper (S) Folgendes umfasst:
Zurückziehen eines Kolbenflansch-Halteelements (494) von dem Spritzenkörper (S) bei geöffnetem Ventil; und
Schließen des Ventils nach dem Rückziehschritt.

13. Verfahren nach einem der Ansprüche 1-12, worin die Entfernungs-, Befüllungs- und Wiederaufsetzschritte für jeden Spritzenkörper (S) an einem ersten Ort des Spritzenkörpers (S) durchgeführt werden.

14. Verfahren nach einem der Ansprüche 1-13, worin das Verfahren für jeden Spritzenkörper (S) ferner die Erfassung der Position eines Kolbenendes umfasst, um den Befüllungsschritt zu beenden.

15. Verfahren nach einem der Ansprüche 1-14, worin die in dem Entfernungsschritt entfernte Kappe (C) für jeden Spritzenkörper (S) dieselbe ist wie die Kappe (C), die in dem Wiederaufsetzschritt wiederaufgesetzt wurde.

16. Verfahren nach einem der Ansprüche 1-15, worin das Verfahren für jeden Spritzenkörper (S) ferner Halten der von dem Spritzenkörper (S) entfernten Kappe (C) bis zum Wiederaufsetzen auf einen der Vielzahl von Spritzenkörpern (S) umfasst.

## Revendications

1. Procédé de remplissage d'une pluralité de corps de seringue (S), dans lequel, pour chaque corps de seringue (S) parmi la pluralité de corps de seringue (S), le procédé comprend les étapes suivantes:
maintenir le corps de seringue (S) dans au moins un support (74);
enlever un capuchon (C) d'une extrémité de distribution du corps de seringue (S) pendant ladite étape de maintien;
remplir le corps de seringue (S) à l'extrémité de distribution de celui-ci après ladite étape d'enlèvement et pendant ladite étape de maintien, ladite étape de remplissage comprenant les étapes suivantes:
interconnecter un appareil de distribution de liquide (420) avec l'extrémité de distribution du corps de seringue (S) d'une façon automatique; et
faire s'écouler un fluide dans le corps de seringue (S) à partir de l'appareil de distribution de fluide (420), à travers l'extrémité de distribution du corps de seringue (S), en attirant le fluide dans le corps de seringue (S) par le retrait d'un plongeur (P) par rapport au corps de seringue (S); et
remettre en place un capuchon (C) enlevé lors de l'étape d'enlèvement de l'un parmi ladite pluralité de corps de seringue (S) sur l'extrémité de distribution du corps de seringue (S) après ladite étape de remplissage et pendant ladite étape de maintien.

2. Procédé selon la revendication 1, dans lequel, pour chaque corps de seringue (S), le procédé comprend en outre les étapes suivantes:
placer ledit capuchon (C) sur ladite extrémité de distribution du corps de seringue (S) avant ladite étape de maintien; et
stériliser le corps de seringue (S) après ladite étape de placement et avant ladite étape de maintien.

3. Procédé selon la revendication 2, dans lequel lesdites étapes de placement et de stérilisation sont accomplies à un premier endroit, et lesdites étapes de maintien, d'enlèvement, de remplissage et de remise en place sont accomplies à un deuxième endroit distant dudit premier endroit, et dans lequel, pour chaque corps de seringue (S), le procédé comprend en outre les étapes suivantes:
emballer ledit corps de seringue (S) dans un contenant (68) à un premier endroit après ladite étape de placement et avant ladite étape de maintien; et
déballer ledit corps de seringue (S) dudit contenant (68) audit deuxième endroit avant ladite étape de maintien.

4. Procédé selon l'une quelconque des revendications 1 à 3, dans lequel le procédé comprend en outre les étapes suivantes:
positionner ladite pluralité de corps de seringue (S) dans une orientation prédéterminée avant ladite étape de maintien; et
interconnecter ladite pluralité de corps de seringue positionnés (S) dans ladite orientation prédéterminée avant ladite étape de maintien.

5. Procédé selon la revendication 4, dans lequel le procédé comprend en outre la disposition de ladite pluralité de corps de seringue interconnectés (S) dans ladite orientation prédéterminée dans une pluralité dudit au moins un support (74) afin d'exécuter au moins une parmi ladite étape de maintien, ladite étape d'enlèvement, ladite étape de remplissage et ladite étape de remise en place.

6. Procédé selon l'une quelconque des revendications 1 à 3, dans lequel le procédé comprend en outre la disposition de ladite pluralité de corps de seringue (S) dans une pluralité dudit au moins un support (74) afin d'exécuter au moins une parmi ladite étape de maintien, ladite étape d'enlèvement, ladite étape de remplissage et ladite étape de remise en place.

7. Procédé selon l'une quelconque des revendications 5 à 6, comprenant en outre le déplacement de ladite pluralité d'au moins un support (74) le long d'un chemin prédéterminé pendant ladite exécution d'au moins une parmi ladite étape de maintien, ladite étape d'enlèvement, ladite étape de remplissage et ladite étape de remis en place.

8. Procédé selon la revendication 7, dans lequel ladite pluralité dudit au moins un support (74) est située sur un élément de support (72), et dans lequel ladite étape de déplacement comprend la mise en rotation dudit élément de support (72).

9. Procédé selon la revendication 7, dans lequel une pluralité de positions de travail est située le long dudit chemin prédéterminé, et dans lequel le procédé comprend en outre la disposition de ladite pluralité de corps de seringue (S) en série à une pluralité de positions de travail afin d'accomplir ladite exécution d'au moins une parmi ladite étape de maintien, ladite étape d'enlèvement, ladite étape de remplissage et ladite étape de remise en place.

10. Procédé selon l'une quelconque des revendications 1 à 9, dans lequel chacun parmi ladite pluralité de corps de seringue (S) comprend un cylindre (B) et un plongeur (P) qui est disposé d'une façon coulissante dans une extrémité du cylindre (B), et dans lequel le capuchon (C) couvre et protège l'extrémité de distribution du corps de seringue (S) avant l'étape d'enlèvement afin de maintenir un volume interne propre dans le corps de seringue (S).

11. Procédé selon l'une quelconque des revendications 1 à 10, dans lequel, pour chaque corps de seringue (S), l'étape d'enlèvement comprend les étapes suivantes:
engager de façon retenue ledit capuchon (C) dans un dispositif de retenue (160); et
déplacer au moins un parmi ledit élément de retenue (160) et ledit au moins un support (74) d'une façon automatique afin d'affecter le déplacement relatif entre le capuchon (C) et l'extrémité de distribution du corps de seringue (S), et
dans lequel, pour chaque corps de seringue (S), l'étape de remplacement comprend les étapes suivantes:
engager de façon retenue ledit un desdits capuchons (C) dans un dispositif de retenue (160); et
déplacer ledit support (74) le long d'un chemin prédéterminé afin d'insérer ledit un desdits capuchons (C) dans le corps de seringue (S).

12. Procédé selon l'une quelconque des revendications 1 à 11, dans lequel ledit appareil de distribution de liquide (420) comprend une soupape, et dans lequel, pour chaque corps de seringue (S), l'étape de remplissage comprend les étapes suivantes:
retirer un élément de retenue de bride de plongeur (494) à l'écart du corps de seringue (S) avec la soupape ouverte; et
fermer la soupape après l'étape de retrait.

13. Procédé selon l'une quelconque des revendications 1 à 12, dans lequel, pour chaque corps de seringue (S), lesdites étapes d'enlèvement, de remplissage et de remise en place sont accomplies à un premier endroit du corps de seringue (S).

14. Procédé selon l'une quelconque des revendications 1 à 13, dans lequel, pour chaque corps de seringue (S), le procédé comprend en outre la détection de la position d'une extrémité de plongeur de celui-ci afin de terminer ladite étape de remplissage.

15. Procédé selon l'une quelconque des revendications 1 à 14, dans lequel, pour chaque corps de seringue (S), le capuchon (C) qui est enlevé lors de l'étape d'enlèvement est le même que le capuchon (C) qui est remis en place lors de ladite étape de remise en place.

16. Procédé selon l'une quelconque des revendications 1 à 15, dans lequel, pour chaque corps de seringue (S), le procédé comprend en outre le maintien du capuchon (C) enlevé du corps de seringue (S) jusqu'à ce qu'il soit remis en place sur l'un parmi la pluralité de corps de seringue (S).
